Europäisches Patentamt

European Patent Office

Office européen des brevets

(19)

(11) EP 0 583 276 B1

# (12) EUROPEAN PATENT SPECIFICATION

(45) Date of publication and mention
of the grant of the patent:
**27.11.1996 Bulletin 1996/48**

(21) Application number: **92908962.1**

(22) Date of filing: **30.04.1992**

(51) Int Cl.6: **D04H 1/54**, A61L 15/00

(86) International application number:
**PCT/GB92/00792**

(87) International publication number:
**WO 92/19802 (12.11.1992 Gazette 1992/28)**

(54) **ALGINATE FABRIC, ITS USE IN WOUND DRESSINGS AND SURGICAL HAEMOSTATS AND A PROCESS FOR ITS MANUFACTURE**

ALIGNATE-STOFF ANGEWENDET ALS VERBANDMATERIAL UND ALS HAEMOSTATISCHES CHIRURGISCHES MATERIAL UND VERFAHREN ZUR HERSTELLUNG

TISSU D'ALGINATE, UTILISATION DANS LES BANDAGES POUR BLESSURES ET LES HEMOSTATIQUES CHIRURGICAUX ET PROCEDE DE FABRICATION

(84) Designated Contracting States:
**AT BE CH DE DK ES FR GB GR IT LI LU NL SE**

(30) Priority: **01.05.1991 GB 9109367**

(43) Date of publication of application:
**23.02.1994 Bulletin 1994/08**

(73) Proprietor: **E.R. SQUIBB & SONS, INC.
Princeton, NJ 08540 (US)**

(72) Inventors:
 • MAHONEY, Peter Michael John, C.V. Lab. Ltd.
  HampshireGU11 1LD e (GB)
 • FENTON, John Charles, C.V. Laboratories Ltd.
  Hampshire GU11 1LDL (GB)
 • KEYS, Allison Frances, C.V. Laboratories Ltd.
  Hampshire GU11 1LD (GB)

(74) Representative: **Mays, Julie et al
Bristol-Myers Squibb Company,
Patent Department,
Swakeleys House,
Milton Road
Ickenham, Uxbridge UB10 8NS (GB)**

(56) References cited:
EP-A- 0 072 680          EP-A- 0 160 560
EP-A- 0 285 707          EP-A- 0 344 913
WO-A-80/02300           WO-A-89/12471
FR-A- 2 267 794          FR-A- 2 268 892
GB-A- 1 379 158

## Description

The present invention relates to an alginate fabric. More particularly, the invention concerns a wound dressing or surgical haemostat formed from a non-woven fabric of alginate fibres which has high absorbency and good integrity and to a process for the preparation of such a fabric.

Alginate fibres have been known for some time as being useful in the preparation of surgical dressings. For example, United Kingdom Patent No. 653341, published in 1951, describes surgical dressings formed from fibres of calcium alginate.

Various types of dressing formed from fabrics comprising alginate fibres are known. For example, a surgical dressing comprising a carded web of layered alginate fibres is known; and Kaltostat haemostatic wound dressing is a carded and needle-tacked web of alginate fibres.

During surgery, it is necessary to remove blood from the wound in order to afford the surgeon a clear view. For use in surgery, where a wound is bleeding freely, the ideal dressing will be haemostatic and will have good structural integrity, high absorbency and a high liquid retention capacity. Good integrity is particularly important for a dressing for use in surgery since it must be possible to lift the whole dressing away from the wound when saturated in blood. High absorbency means that an efficient uptake of exudate or blood, together with its associated toxins and other undesirable matter, can be achieved. A high liquid retention capacity means that blood is retained within the dressing when it is removed from the wound and dripping of blood is minimised.

Conventionally this is achieved by providing absorbent material in the form of cotton or cotton and viscose gauze. While such materials have good absorbency, they have low liquid-retention capabilities. There is therefore a risk when such dressings are used that surrounding areas will be contaminated with blood, and this risk has assumed particular importance in view of current concern regarding infectious diseases such as Aids and hepatitis B.

Furthermore, the gauze dressings currently used in surgery are not haemostatic, that is to say, they do not inhibit bleeding.

Presently available haemostatic alginate dressings are not suitable for use in surgery because they have insufficient structural integrity such that they tend to disintegrate.

EP-A-0344913 describes an alginate wound dressing of an integrity alleged to be such as to enable it to be lifted in one piece from a wound even when saturated with blood or other saline fluids. Briefly, the wound dressing provided by EP-A-0344913 comprises a non-woven fabric of alginate staple fibres, the fabric being substantially free from any adhesive binder or of interfusing of fibres at their crossing points. The required integrity is imparted to the dressing fabric of EP-A-0344913 by subjecting the non-woven web of staple alginate fibres to a hydraulic entanglement procedure which preferably comprises hydroentanglement.

Whilst the wound dressing provided by EP-A-0344913 is of good integrity, it does not have sufficiently high absorbency and liquid-retension capacity to be useful during surgery.

United States Patent no. 3,937,223 discloses a haemostatic surgical felt comprising fibres of a polymer having glycolic acid ester linkages and which is completely absorbed when enclosed in living tissue. The felt has partially compressed heat embossed surfaces. This embossing is said to cause a reduction in the rate of penetration of blood. US Patent no. 3,937,223 contains no suggestion that its teaching could be extended to dressings prepared from different types of fibres. In particular, there is no suggestion that alginates could be used.

United States Patent no. 4,128,612 (divided from above-mentioned US 3,937,223) relates to a process for the manufacture of a haemostatic surgical felt having a textured and embossed surface. Briefly, the process includes the steps of randomly air laying fibres of a living tissue absorbable polymer into a mat and heating and compressing the mat so as to emboss it. US Patent no. 4,128,612 stresses the random formation of the mat and suggests that orientation of the fibres is to be avoided. There is no indication that the process described in the US Patent no. 4,128,612 could be applied to alginate fibres or that a layered mat of fibres could be used.

We have now found that dressings can be prepared from alginate fibres which fulfill the abovementioned criteria for a surgical haemostat.

The requisite good structural integrity can be imparted to the alginate dressing by firmly joining together layers of alginate material at a plurality of points to produce a composite fabric. This may be achieved, for example, by stitching, or, preferably, by calendering.

We have surprisingly found that by embossing regions of the layered alginate fabric, for example, by stitching or calendering, a haemostatic material having good integrity is produced without substantially affecting the absorbency or liquid retention properties of the fabric.

The present invention accordingly provides a non-woven fabric suitable for use as a surgical haemostat comprising layers of alginate staple fibres, which layers are joined together at a plurality of points throughout the fabric whereby an embossed pattern is produced on at least one of the major external surfaces of the fabric. It is necessary that the embossed areas of the fabric should be of sufficient extent to impart integrity, but should not be so extensive that the absorbency of the finished fabric is adversely affected. Suitable ratios for embossed:unembossed area are in a range of 1:1 to 1:9. Preferably the ratio of embossed:unembossed area will be in the region of 1:4.

The present invention provides a non-woven fabric suitable for use as a surgical haemostat comprising layers of alginate staple fibres which layers are joined together at a plurality of points throughout the fabric whereby an embossed pattern is produced on at least one of the major external surfaces of the fabric wherein the ratio of embossed:unembossed area is in the range of 1:1 to 1:9.

The present invention further provides a non-woven fabric suitable for use as a surgical haemostat comprising layers of alginate staple fibres, which layers are joined together at a plurality of points throughout the fabric by stitching or calendering whereby an embossed pattern is produced on at least one of the major external surfaces of the fabric.

In a preferred aspect, the present invention provides a calendered, non-woven, fabric comprising layers of alginate staple fibres which fabric bears an embossed pattern on at least one of its major external surfaces.

Preferably both of the major external surfaces of the fabric according to the invention will bear an embossed pattern.

The haemostatic action of alginates is believed to be related to calcium ion release. On contact with blood or other saline fluids, calcium ions are released and exchanged for sodium ions. Calcium ions interact with the blood clotting cascade mechanism, resulting in clot formation.

We have now found that the degree of calcium ion release from alginate material can be controlled. This means that dressings can be produced to give the appropriate degree of calcium ion release for differing applications.

The degree of calcium ion release can be controlled by selecting an appropriate alginate source material and/or by treating the alginate so as to enhance its calcium ion release properties.

Suitable alginates for use in the invention include both water-soluble and water-insoluble alginates, but will preferably be water-insoluble alginates. A particular water-insoluble alginate for use in the invention is calcium alginate. Nevertheless, the calcium alginate may advantageously contain up to 1.5% by weight of sodium ions. Preferably 99% calcium alginate fibres will be used.

Alginic acid is a linear polymer comprising D-mannuronic acid and L-guluronic acid, the relative proportions of which vary with botanical source and state of maturation of the source plant. Alginates which comprise a high proportion of guluronic acid are known as high G alginates, and alginates which comprise a low proportion of guluronic acid are known as low G alginates.

Both high G alginates and low G alginates are suitable for use in the present invention.

High G alginates chelate calcium more strongly than do low G alginates. Thus, for applications where a high degree of calcium ion release is required, a low G alginate may be selected. For other applications, a high G alginate may be preferred. High G alginate may be obtained from, for example, laminaria hygerborea, and is commercially available as, for example, LF 10/60 from Protan Ltd. Low G alginate may be obtained from, for example, ascophyllum nodosum, and is commercially available as, for example, Lamitex (Protan Ltd.) and Manucol DM (Kelco).

We have now surprisingly found that calcium ion release from any particular alginate can be increased if the alginate material is treated with an oxidising agent. A particularly suitable oxidising agent for this purpose is hypochlorite ion.

According to a further or alternative aspect, the present invention provides a method of enhancing calcium ion release from alginate material which comprises treating the material with an oxidising agent.

For some surgical applications, it is desirable to provide means for detecting the presence of the dressing in the patient's body. Advantageously, X-ray detectable material may be incorporated into the dressing for this purpose.

We have further found that X-ray detectable material can be incorporated into the embossed fabric.

Thus the invention further provides a non-woven fabric suitable for use as a surgical haemostat comprising layers of alginate staple fibres, which layers are joined together at a plurality of points throughout the fabric whereby an embossed pattern is produced on at least one of the major external surfaces of the fabric, and containing X-ray detectable material.

X-Ray detectable material for use in the fabrics of the present invention may take the form of, for example, yarn, ribbon, tape or strip. It is important that the X-ray detectable material is soft and pliable. X-Ray opaque materials include, for example, barium sulphate, tungsten and bismuth. A number of carrier materials for the X-ray opaque material are commercially available, including PVC, polyurethane (PU) and silicon.

The preferred X-ray detectable material for use in the present invention is one or more PVC X-ray detectable strips containing barium sulphate.

Advantageously the X-ray detectable material may be at least partially bound to the alginate fabric. Where the carrier material comprises a thermally fusable material, such as a plastic, for example PVC or PU, bonding of the X-ray detectable material to the alginate fabric may be achieved by partially melting the carrier material before or during insertion of the X-ray detectable material into the fabric, or, preferably, during the embossing process.

Calendering is a process whereby a web of material is compressed, usually between heated rollers.

In order to produce an embossed pattern, at least one the rollers of the calender must have a textured surface. For example, one or both rollers may have a plurality of either engravings or, preferably, raised portions. Conveniently, only one of the rollers has a textured surface.

The effect of calendering with a textured roller is that areas of the fabric surface are compressed to different extents such that areas which are compressed to a lesser extent become raised relative to areas which undergo greater compression, resulting in an embossed pattern.

Even if only one of the rollers of the calender has a textured surface, an embossed pattern may be produced on both major external surfaces of the finished fabric. This is because the pressure of the textured roller on the surface of the fabric it contacts tends to cause a reverse or "negative" of the embossed pattern produced on that surface to be produced on the opposite major external surface of the fabric. This "negative" pattern is considerably less pronounced than the pattern produced by direct embossment.

Where a fabric having an embossed pattern on both of its major external surfaces is referred to herein it is to be understood that a fabric having one directly embossed surface and one surface bearing a "negative" of the embossed pattern is included.

The quality of the embossed pattern is dependent on the temperature, pressure and speed of the rollers during the calendering process. If the pattern is not firmly embossed the calendered regions of the fabric tend to revert and in a relatively short time the embossed effect is lost. We have found that best results are achieved using a roller speed of 1 to 40 metres per minute, preferably 1 to 10 metres per minute, more preferably 3 metres per minute at a temperature of about 80°C to about 210°C, preferably 130°C and a pressure of 1 ton to 15 tons, preferably approximately 6 tons.

The wind up tension should be kept to a minimum. Over tensioning the web as it is wound up will tend to stretch the material as it leaves the calender nip. This results in reduced absorbency of the final product.

The present invention therefore provides a process for the preparation of a fabric which process comprises the steps of:

(a) providing a first layer of alginate fibres;
(b) arranging a further layer of alginate fibres on the first layer to form a web;
(c) joining the layers together at a plurality of points throughout the web;
(d) calendering regions of the web so as to produce an embossed pattern on at least one major external surface of the fabric wherein the ratio of embossed:unembossed area is in a range of 1:1 to 1:9. Preferably the calendering process will be conducted at a temperature of from 80°C to 210°C and a pressure of from 1 ton to 15 tons.

Preferably the web of non-woven layered alginate fibres is produced by cross-lapping. Cross-lapping is a process whereby a web is built up by the sequential laying of layers of fibres on another until a web of the desired weigh is achieved. Conveniently, fibres in alternate layers are oriented orthogonally to each other, but this is not essential. Preferably the layers of alginate fibres produced by cross-lapping are tacked together using a needle punching procedure.

According to a further or alternative aspect the present invention provides a process for incorporation of one or more X-ray detectable strips into an alginate fabric which process comprises the additional step carried out before step (c) of positioning one or more X-ray detectable strips between webs of alginate fibres as produced in steps (a) and (b).

In order to avoid damage to the X-ray detectable strip during calendering, at least one of the top and bottom rollers of the calender is provided with one or more grooves. Each X-ray detectable strips in the fabric is aligned so as to coincide with a groove in the roller. Alignment can be achieved manually, or automatically or semi-automatically using, for example, an optical, infra red or electronic control system.

The fabric prepared by the process according to he invention typically has absorbency in the region of 10.5 times its own weight and a water-retention capacity in the region of 9.5 times its own weight. This compares with absorbency in the region of 6.5 times its own weight and a water-retention capacity in the region of 6.5 times its own weight for conventional surgical gauze.

While the fabric according to the present invention is particularly suitable for use as a surgical haemostat, it will be appreciated that it can also advantageously be employed as a wound dressing for non-surgical applications. The fabric according to the present invention is also particularly suitable for use in dentistry.

As will be appreciated, the alginate fabric according to the invention can suitably be manufactured in a range of basis weights, typically from about $120g/m^2$ to about $360g/m^2$, preferably from about $160g/m^2$ to about $240g/m^2$, more preferably, about $180g/m^2$. The basis weight of a given fabric will generally be dependent upon the use, for example as a wound dressing or surgical haemostat, to which the fabric is to be put. By way of example, a basis weight in the region of $120g/m^2$ is indicated for a moderately exuding wound whereas a basis weight in the region of $280g/m^2$ is indicated for a heavily exuding or freely bleeding wound.

In another aspect, the present invention provides a wound dressing or surgical haemostat comprising a non-woven fabric comprising layers of alginate staple fibres which layers are joined together at a plurality of points throughout the fabric whereby an embossed pattern is produced, optionally containing X-ray detectable material.

In a further aspect, the present invention provides a method of treating a mammalian subject undergoing surgery, which method comprises applying to the site of surgery a surgical haemostat according to the present invention.

In a still further aspect, the present invention provides a method of treating a mammalian subject having a wound, which method comprises applying to the wound a wound dressing according to the present invention.

As used herein the term "wound" includes cut, sore, ulcer, blister, burn, rash or any other lesion or area of troubled skin.

The wound dressings or surgical haemostats formed from the alginate fabric according to the present invention will advantageously be conventional dressings well known in the art. Examples of suitable dressings include bandages, adhesive strip dressings, island dressings, pads of various kinds, surgical sponges and packs, ward dressings, and such articles as tampons which may, for example, be impregnated with an antifungal agent such as miconazole for the treatment of candidal vaginitis (vaginal thrush). Such dressings may conveniently be prepared by standard methods known from the art.

The dressings in accordance with the present invention will conveniently be packaged in a hermetically-sealed envelope and sterilised, e.g. with ethylene oxide or by gamma-irradiation.

The following non-limiting Example is intended to illustrate the present invention.

EXAMPLE 1

Calcium alginate fibre was prepared as described in Preparation 1 of WO-A-89/12471, crimped, staple cut and converted to non-woven fabric by a conventional carding, cross-lapping and needle punching technique.

The calender is heated to 130°C. Web (basis weight 180g/m$^2$) is loaded to the feed roller. A core is loaded to the wind up and locked in position. The web is laid on the top surface of the fabric leader and the X-ray detectable strip aligned with the grooves in the calender. The material is passed through the calender nip and collected on the wind up roller.

TEST METHOD

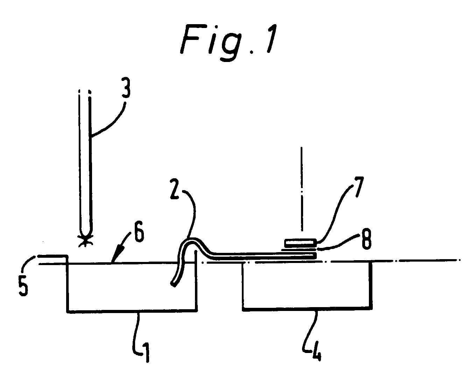

The apparatus used in the determination of absorbency is depicted in Fig. 1, and consists of water bath 1 containing a 0.9% (w/w) aqueous saline solution, absorbent strip 2, burette 3, top-pan balance 4 and overflow 5.

The thickness of the absorbent strip 2 is substantially equivalent to that of the dressing 7. The filter paper 8 has substantially the same planar dimensions as the dressing 7 but not necessarily the same thickness.

The apparatus is set up with the surface 6 of the saline solution level with the top surface of the top-pan balance 4. The flow of liquid from the burette 3 is then adjusted to approximately 1.5 ml per minute. The absorbent strip 2 is then saturated and placed between the bath 1 and the balance 4, as depicted in Fig. 1. The balance 4 is then tared. A weighed dressing 7 and filter paper 8 (cut to size) is positioned as depicted in Fig.1.

After six minutes the weight shown on the balance 4 is recorded. The dressing 7 and filter paper 8 are then removed and any residual weight on the balance 4 noted.

Absorbency is determined on the basis of the following equation:

```
wt. of liquid
absorbed       =

total wt.      dry wt.      wt. of satd.     residual wt.
on balance     dressing  +  filter paper  +  on balance
```

RESULTS

Using the test method described above, the absorbencies of the fabric according to the invention, and of commercially available surgical gauze (4 layers), were determined and compared. In the former case twelve samples, and in the latter case six samples, of the fabric were taken and an average value for the absorbency was calculated. The results obtained were as follows:

TABLE I

| Alginate fabric of Example 1 | | |
|---|---|---|
| Wt. of dressing (g) | Wt. of saline absorbed (g) | Wt. of saline absorbed per gram of dressing (g) |
| 1.14 | 10.21 | 10.61 |
| 0.87 | 8.20 | 9.43 |
| 0.84 | 8.31 | 9.90 |
| 1.20 | 13.68 | 11.40 |
| 1.16 | 12.18 | 10.50 |
| 0.98 | 10.61 | 10.82 |
| 0.95 | 10.35 | 10.89 |
| 1.23 | 13.18 | 10.71 |
| 1.18 | 12.20 | 10.33 |
| 1.31 | 14.13 | 10.78 |
| 1.12 | 12.14 | 10.83 |
| 1.06 | 11.20 | 10.56 |

TABLE II

| GAUZE | | |
|---|---|---|
| Wt. of dressing (g) | Wt. of saline absorbed (g) | Wt. of saline absorbed per gram of dressing (g) |
| 1.04 | 6.85 | 6.58 |
| 1.05 | 6.24 | 5.94 |
| 0.89 | 6.01 | 6.75 |
| 0.92 | 6.37 | 6.92 |
| 0.94 | 5.55 | 5.90 |
| 0.94 | 6.06 | 6.45 |

From Table I above, it can be calculated that the average absorbency of the alginate fabric of Example I is 10.56g of saline per gram of dressing; whereas, from Table II, the average absorbency of commercial surgical gauze (4 layers) can be calculated to be 6.42g of saline per gram of dressing.

RETENTION STUDIES

Samples of fabric according to the invention, and of commercially available surgical gauze (4 layers), were cut to size (approximately 5 x 10 cm) and placed in a dish. Sufficient calf serum was added to just cover the dressings. After 6 minutes the dressings were carefully removed, held on an edge and allowed to drip. The weight of liquid retained and that lost were recorded. The results obtained were as follows:

TABLE III

| Alginate fabric of Example 1 | | |
|---|---|---|
| Wt. of dressing (g) | Wt. of serum retained (g) | Wt. of serum lost (g) |
| 0.98 | 9.38 | 1.21 |
| 0.82 | 8.38 | 0.91 |
| 1.03 | 8.78 | 0.84 |
| 0.92 | 8.53 | 1.06 |
| 0.99 | 9.26 | 1.21 |
| 0.84 | 9.18 | 0.73 |
| 0.97 | 9.01 | 1.33 |
| 1.02 | 9.20 | 0.91 |
| 1.13 | 10.10 | 0.57 |

TABLE III   (continued)

| Alginate fabric of Example 1 | | |
|---|---|---|
| Wt. of dressing (g) | Wt. of serum retained (g) | Wt. of serum lost (g) |
| 1.01 | 9.78 | 0.83 |

TABLE IV

| GAUZE | | |
|---|---|---|
| Wt. of dressing (g) | Wt. of serum retained (g) | Wt. of serum lost (g) |
| 0.64 | 4.06 | 2.58 |
| 0.82 | 5.40 | 3.12 |
| 0.84 | 5.26 | 3.46 |
| 0.93 | 6.21 | 3.81 |
| 1.12 | 7.13 | 4.71 |
| 0.76 | 4.73 | 3.05 |

From Table III above, it can be calculated that the average retention capacity of the alginate fabric of Example I is 9.48g of serum per gram of dressing; whereas, from Table IV, the average retention capacity of commercial surgical gauze can be calculated to be 6.4g of serum per gram of dressing.

## Claims

1. A non-woven fabric of alginate staple fibres characterised in that the alginate fibres are arranged in layers which layers are joined together at a plurality of points throughout the fabric whereby an embossed pattern is produced on at least one of the major external surfaces of the fabric wherein the ratio of embossed:unembossed area is in the range of 1:1 to 1:9.

2. A fabric as claimed in claim 1 wherein the ratio of embossed:unembossed area is about 1:4.

3. A fabric as claimed in claim 1 or claim 2 wherein the embossed pattern is produced by calendering.

4. A fabric as claimed in any preceding claim wherein both major external surfaces bear an embossed pattern.

5. A fabric as claimed in any preceding claim wherein the alginate staple fibres comprise 99% calcium alginate.

6. A fabric as claimed in any preceding claim containing X-ray detectable material.

7. A fabric as claimed in claim 6 wherein the X-ray detectable material is one or more PVC strips containing barium sulphate.

8. A fabric as claimed in claim 6 or claim 7 wherein the X-ray detectable material is at least partially bound to the alginate fabric.

9. A fabric as claimed in any preceding claim having a basis weight in the range of about 120g/m$^2$ to about 360g/m$^2$.

10. A fabric as claimed in claim 9 having a basis weight in the range of about 160g/m$^2$ to about 240g/m$^2$.

11. A non-woven fabric suitable for use as a surgical haemostat having a basis weight of about 180g/m$^2$, which fabric comprises layers of 99% calcium alginate staple fibres, which layers are joined together at a plurality of points throughout the fabric by calendering whereby an embossed pattern is produced on both major external surfaces of the fabric wherein the ratio of embossed:unembossed area is about 1:4, and which fabric contains at least one PVC strip containing barium sulphate.

**12.** A fabric as claimed in claim 12 wherein the PVC strip(s) is (are) partially bonded to the alginate fabric.

**13.** A process for the preparation of a fabric as claimed in any of claims 1 to 12 which process comprises the steps of:

    (a) providing a first layer of alginate fibres;
    (b) arranging a further layer of alginate fibres on the first layer to form a web;
    (c) joining the layers together at a plurality of points throughout the web;
    (d) calendering regions of the web so as to produce an embossed pattern on at least one major external surface of the fabric wherein the ratio of embossed:unembossed area is in a range of 1:1 to 1:9.

**14.** A process as claimed in claim 13 wherein the calendaring in step (d) is conducted at a temperature of from 80°C to 210°C and a pressure of from 1 ton to 15 tons.

**15.** A process as claimed in claim 13 or claim 14 comprising the additional step before step (c) of positioning one or more X-ray detectable strips between webs of alginate fibres as produced in steps (a) and (b).

**16.** A process as claimed in claim 15 wherein at least the top and bottom rollers of the calender are provided with one or more grooves and each X-ray detectable strip is aligned so as to coincide with a groove in the rollers(s).

**17.** A process as claimed in claim 15 or claim 16 which further comprises the step of at least partially bonding the X-ray detectable strip to the alginate fabric.

**18.** A process as claimed in any of claims 13 to 17 which further comprises the step of treating the alginate fabric with an oxidising agent.

**19.** A process as claimed in claims 18 wherein the oxidising agent is a hypochlorite ion.

**20.** A surgical haemostat comprising a fabric as claimed in any of claims 1 to 12.

**21.** A wound dressing comprising a fabric as claimed in any of claims 1 to 12.

**22.** The use of a fabric as claimed in any of claims 1 to 21 as a surgical haemostat.


**Patentansprüche**

**1.** Vlies aus Alginatstapelfasern, dadurch gekennzeichnet, daß die Alginatfasern in Schichten angeordnet sind. wobei die Schichten an einer Vielzahl von Punkten überall im Vlies miteinander verbunden sind, wodurch ein geprägtes Muster auf wenigstens einer der Hauptaußenflächen des Vlieses erzeugt wird, und wobei das Verhältnis von geprägtem zu ungeprägtem Bereich in der Größenordnung von 1:1 bis 1:9 liegt.

**2.** Vlies nach Anspruch 1. wobei das Verhältnis von geprägtem zu ungeprägtem Bereich etwa 1:4 beträgt.

**3.** Vlies nach Anspruch 1 oder 2, wobei das geprägte Muster durch Kalandrieren hergestellt wird.

**4.** Vlies nach einem der vorstehenden Ansprüche, wobei beide Hauptaußenflächen ein geprägtes Muster tragen.

**5.** Vlies nach einem der vorstehenden Ansprüche, wobei die Alginatstapelfasern 99% Calciumalginat umfassen.

**6.** Vlies nach einem der vorstehenden Ansprüche. das ein durch Röntgenstrahlen detektierbares Material enthält.

**7.** Vlies nach Anspruch 6, wobei das durch Röntgenstrahlen detektierbare Material ein oder mehrere PVC-Streifen sind, die Bariumsulfat enthalten.

**8.** Vlies nach einem der Ansprüche 6 oder 7, wobei das durch Röntgenstrahlen detektierbare Material wenigstens teilweise mit dem Alginatstoff verbunden ist.

**9.** Vlies nach einem der vorstehenden Ansprüche mit einem Grundgewicht im Bereich von etwa 120 g/m$^2$ bis etwa

360 g/m$^2$.

10. Vlies nach Anspruch 9 mit einem Grundgewicht im Bereich von etwa 160 g/m$^2$ bis etwa 240 g/m$^2$.

11. Vlies, das zur Verwendung als chirurgisches Hämostatikum geeignet ist, mit einem Grundgewicht von etwa 180 g/m$^2$, wobei das Vlies Schichten aus 99%igen Calciumalginatstapelfasern umfaßt, und die Schichten an einer Vielzahl von Punkten überall im Vlies durch Kalandrieren miteinander verbunden sind, wodurch ein geprägtes Muster auf beiden Hauptaußenflächen des Vlieses erzeugt wird, wobei das Verhältnis von geprägtem zu ungeprägtem Bereich etwa 1:4 beträgt, und wobei das Vlies wenigstens einen PVC-Streifen umfaßt, der Bariumsulfat enthält.

12. Vlies nach Anspruch 11, wobei der(die) PVC-Streifen teilweise an den Alginatstoff gebunden ist(sind).

13. Verfahren zur Herstellung eines Vlieses nach einem der Ansprüche 1 bis 12, wobei das Verfahren die Schritte umfaßt:

(a) Bereitstellen einer ersten Schicht aus Alginatfasern,
(b) Anordnen einer weiteren Schicht aus Alginatfasern auf der ersten Schicht, wodurch eine Bahn erzeugt wird,
(c) Verbinden der Schichten an einer Vielzahl von Punkten überall in der Bahn,
(d) Kalandrieren von Bereichen der Bahnen, wodurch auf wenigstens einer der Hauptaußenflächen des Vlieses ein geprägtes Muster erzeugt wird, wobei das Verhältnis von geprägtem zu ungeprägtem Bereich in der Größenordnung von 1:1 bis 1:9 liegt.

14. Verfahren nach Anspruch 13, wobei das Kalandrieren in Schritt (d) bei einer Temperatur von 80 bis 210°C und unter einem Druck von 1 bis 15 Tonnen durchgeführt wird.

15. Verfahren nach einem der Ansprüche 13 oder 14, umfassend vor Schritt (c) die zusätzlichen Schritte: Einlegen eines oder mehrerer durch Röntgenstrahlen detektierbaren Streifen(s) zwischen die Bahnen aus Alginatfasern, die in den Schritten (a) und (b) erzeugt wurden.

16. Verfahren nach Anspruch 15, wobei wenigstens die Ober- und Unterwalzen des Kalanders mit einer oder mehreren Riffelungen versehen sind, und jeder der durch Röntgenstrahlen detektierbaren Streifen so ausgerichtet ist, daß er entlang der Riffelung der Walze(n) ausgerichtet ist.

17. Verfahren nach einem der Ansprüche 15 oder 16, ferner umfassend den Schritt: wenigstens teilweises Verbinden des durch Röntgenstrahlen detektierbaren Streifens mit dem Alginatvlies.

18. Verfahren nach einem der Ansprüche 13 bis 17. ferner umfassend den Schritt: Behandeln des Alginatvlieses mit einem Oxidationsmittel.

19. Verfahren nach Anspruch 18, wobei das Oxidationsmittel ein Hypochlorition ist.

20. Chirurgisches Hämostatikum, umfassend ein Vlies nach einem der Ansprüche 1 bis 12.

21. Verbandmaterial, umfassend ein Vlies nach einem der Ansprüche 1 bis 12.

22. Verwendung eines Vlieses nach einem der Ansprüche 1 bis 21 als chirurgisches Hämostatikum.

**Revendications**

1. Tissu non tissé de fibres d'alginate coupées, caractérisé en ce que des fibres d'alginate sont arrangées en couches, lesquelles couches sont jointes ensemble à une pluralité de points au travers du tissu, ce par quoi un motif embouti est produit sur au moins une des surfaces externes principales du tissu, où le rapport de la surface emboutie: non emboutie est dans l'intervalle de 1:1 à 1:9.

2. Tissu selon la revendication 1, dans lequel le rapport de la surface emboutie:non emboutie est d'environ 1:4.

3. Tissu selon la revendication 1 ou la revendication 2, dans lequel le motif embouti est produit par calendrage.

4. Tissu selon l'une quelconque des revendications précédentes, dans lequel les deux surfaces externes principales portent un motif embouti.

5. Tissu selon l'une quelconque des revendications précédentes, dans lequel les fibres coupées d'alginate comprennent 99% d'alginate de calcium.

6. Tissu selon l'une quelconque des revendications précédentes, contenant un matériau détectable aux rayons X.

7. Tissu selon la revendication 6, dans lequel le matériau détectable aux rayons X est une ou plus rayures de PVC contenant du sulfate de baryum.

8. Tissu selon la revendication 6 ou la revendication 7, dans lequel le matériau détectable aux rayons X est au moins partiellement lié au tissu d'alginate.

9. Tissu selon l'une quelconque des revendications précédentes, ayant un poids de base dans l'intervalle d'environ 120 g/m$^2$ à environ 360 g/m$^2$.

10. Tissu selon la revendication 9 ayant un poids de base dans l'intervalle d'environ 160 g/m$^2$ à environ 240 g/m$^2$.

11. Tissu non tissé approprié pour utilisation en tant que pansement hémostatique chirurgical ayant un poids de base d'environ 180 g/m$^2$, lequel tissu comprend des couches de fibres coupées d'alginate de calcium à 99%, lesquelles couches sont jointes ensemble à une pluralité de points au travers du tissu par calandrage, ce par quoi un motif embouti est produit sur les deux surfaces externes principales du tissu, où le rapport de la surface emboutie:non emboutie est d'environ 1:4, et lequel tissu comprend au moins une rayure de PVC contenant du sulfate de baryum.

12. Tissu selon la revendication 12, dans lequel la(les) rayure(s) de PVC est(sont) partiellement liée(s) au tissu d'alginate.

13. Procédé pour la préparation d'un tissu selon l'une quelconque des revendications 1 à 12, lequel procédé comprend les étapes de :

(a) fourniture d'une première couche de fibres d'alginate;
(b) arrangement d'une couche supplémentaire de fibres d'alginate sur la première couche pour former une bande;
(c) jonction des couches ensemble à une pluralité de points au travers de la bande;
(d) calendrage des régions de la bande de façon à produire un motif embouti sur au moins une surface externe principale du tissu, où le rapport de la surface emboutie:non emboutie est dans un intervalle de 1:1 à 1:9.

14. Procédé selon la revendication 13, dans lequel le calendrage dans l'étape (d) est mené à une température de 80°C à 210°C et à une pression de 1 tonne à 15 tonnes.

15. Procédé selon la revendication 13 ou la revendication 14 comprenant l'étape additionnelle avant l'étape (c) de positionnement d'une ou plusieurs rayures détectables aux rayons X entre les bandes de fibres d'alginate telles que produites aux étapes (a) et (b).

16. Procédé selon la revendication 15, dans lequel au moins les rouleaux supérieur et inférieur du calendreur sont munis d'une ou plusieurs rainures et chaque bande détectable aux rayons X est alignée de façon à coincider avec une rainure dans le(s) rouleau(x).

17. Procédé selon la revendication 15 ou la revendication 16 qui comprend de plus l'étape de lier au moins partiellement la rayure détectable aux rayons X avec le tissu d'alginate.

18. Procédé selon l'une quelconque des revendications 13 à 17, qui comprend de plus l'étape de traitement du tissu d'alginate avec un agent oxydant.

19. Procédé selon la revendication 18, dans lequel l'agent oxydant est l'ion hypochlorite.

**20.** Pansement hémostatique chirurgical comprenant un tissu selon l'une quelconque des revendications 1 à 12.

**21.** Pansement pour blessure comprenant un tissu selon l'une quelconque des revendications 1 à 12.

**22.** Utilisation d'un tissu selon l'une quelconque des revendications 1 à 21 en tant que pansement hémostatique chirurgical.

# Fig.1